# EUROPEAN PATENT APPLICATION

(11) **EP 4 201 521 A1**
(43) Date of publication of application: **28.06.2023**
(21) Application number: 21217621.8
(22) Date of filing: 23.12.2021
(51) Int. Cl.: B01J 23/889, B01J 23/94, B01J 37/18, B01J 37/08, C10G 2/00

(54) **FISCHER-TROPSCH CATALYST ACTIVATION**

(71) Applicant: BP P.L.C., London SW1Y 4PD (GB); Johnson Matthey Davy Technologies Limited, London EC4A 4AB (GB)
(72) Inventor: The designation of the inventor has not yet been filed
(74) Representative: Hamer, Christopher K.

(57) **Abstract**

The present disclosure relates generally to processes for activating Fischer-Tropsch synthesis catalysts. In particular, the application concerns a process for the activation of a Fischer-Tropsch synthesis catalyst, the process comprising: (i) contacting the catalyst with a first gaseous composition comprising at least 80% N₂ at a pressure in the range of 2 barg to 20 barg at a temperature of no more than 150 °C; (ii) contacting the catalyst with a second gaseous composition comprising at least 80% H₂ to form a H₂/N₂ gaseous composition with a H₂:N₂ molar ratio in the range of 0.2:1 to 2:1, resulting in a pressure in the range of 10 barg to 30 barg; (iii) increasing the temperature to a range of 220 °C to 260 °C; (iv) maintaining the catalyst at the conditions of step (iii) for a hold period in the range of 2 hr to 96 hr.

## Description

### BACKGROUND OF THE DISCLOSURE

### FIELD

The present disclosure relates Fischer-Tropsch processes, specifically to techniques for activating a Fischer-Tropsch catalyst, and uses thereof.

### TECHNICAL BACKGROUND

The conversion of synthesis gas into hydrocarbons by the Fischer-Tropsch process has been known for decades but has historically lagged in performance compared to other hydrocarbon synthesis techniques. The growing importance of alternative energy sources has resulted in renewed interest in the Fischer-Tropsch (FT) process as it allows a direct and environmentally acceptable route to high-quality fuels and feedstock chemicals.

FT processes are known for producing linear hydrocarbons for use in fuels, as well as oxygenates which serve as valuable feedstock chemicals. The hydrocarbon fuel deriving from FT processes is better able to meet increasingly stringent environmental regulations compared to conventional refinery-produced fuels, as FT-derived fuels typically have lower contents of sulfur, nitrogen, and aromatic compounds which contribute to the emission of potent pollutants such as SO₂, NOₓ, and particulates. Alcohols derived from FT processes often have a higher octane rating than hydrocarbons and thus burn more completely, thereby reducing the environmental impact of such a fuel. Alcohols and other oxygenates obtained may also be used as reagents in other processes, such as in the synthesis of lubricants.

A variety of transition metals have been identified to be catalytically active in the conversion of synthesis gas into hydrocarbons and oxygenated derivatives thereof. In particular, cobalt, nickel, and iron have been studied, often in combination with a support material, of which the most common are alumina, silica and carbon.

In the typical preparation of supported cobalt-containing FT synthesis catalysts, a solid support material is contacted with a solution of a soluble cobalt compound, such as cobalt nitrate. The impregnated support is subsequently calcined and/or oxidized to form a cobalt oxide, typically one or more of CoO, CO₂O₃, or CO₃O₄. However, such oxides typically have poor FT catalytic activity and must be reduced to form the preferred catalytically active species of cobalt metal.

Efficient catalyst reduction and activation is an ongoing challenge. In large-scale reactors, competing factors, such as temperature, pressure, GHSV (Gas Hourly Space Velocity), and activation gas composition, must be balanced according to system tolerances, equipment design limitations and energy considerations. Accordingly, there exists a need to develop improved protocols for the activation of Fischer-Tropsch catalysts.

### SUMMARY

The inventors have developed processes to activate Fischer-Tropsch synthesis catalysts with increased energy and material efficiency.

Thus, in one aspect, the present disclosure provides a process for the activation of a process for the activation of a Fischer-Tropsch synthesis catalyst, the process comprising:
(i) contacting the catalyst with a first gaseous composition comprising at least 80% N₂ at a pressure in the range of 2 barg to 20 barg at a temperature of no more than 250 °C;
(ii) introducing a second gas composition to the first gas composition below 175°C where the second gaseous composition comprises at least 80% H₂ to form a H₂/N₂ gaseous composition with a H₂:N₂ molar ratio in the range of 0.2:1 to 2:1, resulting in a pressure in the range of 10 barg to 30 barg;
(iii) increasing the temperature to a range of 220 °C to 260 °C at a ramp rate of no more than 20 °C/hr, and a GHSV in the range of 2000 hr⁻¹ to 8000 hr⁻¹;
(iv) maintaining the catalyst at the conditions of step (iii) for a hold period in the range of 2 hr to 96 hr;
(v) cooling the catalyst to a temperature of no more than 150 °C.

In another aspect, the present disclosure provides for a process as otherwise described herein, wherein the process further comprises, during steps (ii)-(v), monitoring the water concentration of the H₂/N₂ gaseous composition.

Other aspects of the disclosure will be apparent to those skilled in the art in view of the description that follows.

### BRIEF DESCRIPTION OF THE DRAWINGS

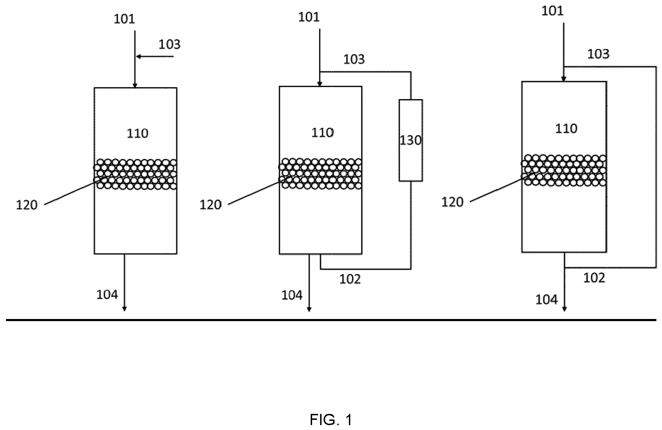

Fig. 1 is a schematic depiction of a reactors suitable for use in the processes described herein.

### DETAILED DESCRIPTION

The present disclosure is concerned with the processes for activating a Fischer-Tropsch catalyst. Typical activation as known in the art involves the use of high temperatures greater than 300 °C and pure hydrogen gas. However, the present inventors have found that such simple activation protocols are poorly suited to large-scale reactors that utilize large amounts of catalyst and reduction gas. At large scale, a compressor capable of handling over 5000 Nm³/m³.h at pressures in excess of 10 barg is utilized. Converted into absolute terms, over 20m3 of catalyst used in a typical reactor or 1000 reactor tubes. Critically, compressors operating at such extreme conditions have narrow tolerance and design windows with regard to GHSV. The compressor is ultimately sized for the Fischer-Tropsch conversion process rather than the very occasional (but equally critical) catalyst activation process. As such these are often designed for 20-50barg of syngas/inerts which have a higher mass that pure hydrogen in activation. This all leads away from an optimal lab activation type condition of low pressure, high hydrogen concentration and high GHSV. Compressors typically direct large-scale conditions, for the FT process where low mass hydrogen is only a part of the syngas feed. During catalyst activation these constraints work against the reduction process with the compressor only operating for reduced hydrogen partial pressures, increased reactor pressure and/or low gas GHSV leading to reduction inhibition and catalyst damage by sintering. Accordingly, the present inventors have found that the temperature, GHSV, reactor pressure, and reduction gas composition must all be carefully controlled relative to each other. Further, catalyst reduction often generates large amounts of water which must be economically removed in order to avoid premature catalyst deactivation.

The present inventors have surprisingly found that high performance preparation of Fischer-Tropsch catalysts can only be found by meeting a balance between activation pressure, temperature, %H₂, and GHSV. On the industrial scale, gases are introduced through the use of a high-capacity compressor. In certain embodiments as otherwise described herein, the compressor has a volumetric capacity of at least 5000 Nm³/m3.hr. The use of such a compressor introduces significant design challenges that are not found in smaller scale investigations. For example, the inventors have found that there is a tension between GHSV, and %H₂: it has been found that high GHSV is required in order to remove excess water produced as a reduction byproduct. However, it is very difficult and inefficient to operate an industrial compressor at high GHSV with a low density gas, such as H₂. The GHSV is challenging to achieve primarily because of the lower pressure needed for activation; but there is also the impact of low molecular weight H₂ gas reducing the developed head of the machine, and thus the flow must also reduce to keep the machine head balanced with the loop pressure drop.

Additionally, using very high H₂ content adds design complexity and cost to the compressor - both from metallurgical issues with high impeller tip speeds leading to high temperatures; and other issues around designing equipment for high hydrogen content, such as seal systems. However, a certain amount of H₂ is required to act as a reactant for the reduction reaction. Accordingly, it is desirable necessary to provide a mixture of N₂ and H₂ as otherwise described herein.

As another example, high GHSV with an industrial compressor is only possible utilizing high pressures. High pressure is also advantageous for driving the reduction reaction through increased H₂ partial pressure. However, high pressure operation leads to ineffective water removal as the inventors have found that high operating pressures can result in trapped water in catalyst pores. Accordingly, the pressure ranges as described herein reflect a critical range that balances these competing factors.

As a further example, temperature must also be carefully controlled. Higher temperatures favor more complete reduction, and also favor water removal. However, above a particular temperature (e.g., in certain embodiments, above 260 °C), the reactor metallurgy can become unstable and prone to damage. Accordingly, keeping a low temperature advantageously avoids the capital expenditure associated with the construction of a reactor that can tolerate extreme temperatures. The mechanical limits are due to the desire to use steam sparging of the shell to generate the reduction pressure. For example a reduction temperature of 250°C corresponds to just under 40 bara saturated pressure which is manageable for the steam drum associated with the FT converter. Higher temperatures would require higher steam pressures and higher pressure rating for shell but would also require thicker tubes to withstand the additional external pressure so would result in increased capex.

Thus, in one aspect, the present disclosure provides a process for the activation of a Fischer-Tropsch synthesis catalyst, the process comprising:
(i) contacting the catalyst with a first gaseous composition comprising at least 80% N₂ at a pressure in the range of 2 barg to 20 barg at a temperature of no more than 250 °C ;
(ii) contacting the catalyst with a second gaseous composition comprising at least 80% H₂ to form a H₂/N₂ gaseous composition with a H₂:N₂ molar ratio in the range of 0.2:1 to 2:1, resulting in a pressure in the range of 10 barg to 30 barg;
(iii) increasing the temperature to a range of 230 °C to 260 °C at a ramp rate of no more than 20 °C/hr, and a GHSV in the range of 2000 hr⁻¹ to 8000 hr⁻¹;
(iv) maintaining the catalyst at the conditions of step (iii) for a hold period in the range of 3 hr and 72 hr;
(v) cooling the catalyst to a temperature of no more than 150 °C.

In another aspect, the present disclosure provides for a process as otherwise described herein, wherein step (i) includes either a stand alone catalyst drying step prior to step (i), or encompasses the drying step with in step (i) as part of the first gaseous composition treatment.

As described in more detail below, a variety of reaction systems can be used in performing the processes described herein, and the person of ordinary skill in the art will adapt conventional systems as necessary. Suitable means for controlling the reactor temperature during activation and start-up can be used, as known by those skilled in the art; including electrical heating, direct heating of the reactor tubes with steam, or indirect heating with steam via an eductor or jet pump arrangement to heat a circulating-water circuit around the reactor tubes. An example of a reaction system for use in performing the processes of the disclosure is shown in schematic view in FIG. 1. Reactor 110 is charged with catalyst 120. Gas (e.g., the first or second gaseous compositions, or combinations thereof) is introduced through inlet 101, and optionally run as a once-through configuration, exiting the system at 104 Unreacted gas are optionally removed through recycle outlet 102, and optionally recycled back into inlet 101 through recycle feed 103 and a moisture-removal dryer 130.

The processes as otherwise described herein may be operated within a Fischer-Tropsch reactor to effect an *in situ* activation. Alternatively, the person of ordinary skill in the art may choose to activate the catalyst in a dedicated reactor and then transfer the activated catalyst, desirably under inert conditions, to a Fischer-Tropsch reactor for conducting Fischer-Tropsch synthesis.

As noted above, the process as otherwise described herein, in certain embodiments, comprises a step of contacting the catalyst with a majority N₂ gas at relatively low temperatures and pressures. Surprisingly, the present inventors have discovered that the initial pressurization of step (i) is advantageously performed with majority nitrogen gas as opposed to a high-H₂ gas. Without wishing to be bound by theory, the inventors have found that pressurizing the reactor with a heavier gas, such as N₂, is much more efficient than pressurization with H₂ or a gas comprising large amounts of H₂. This is because, at the industrial scale, a gas that includes large amounts of H₂ is difficult to pressurize given the constraints of the industrial compressor. Accordingly, the first gaseous composition advantageously comprises a majority N₂ gas. In certain embodiments as otherwise described herein, the first gaseous composition comprises at least 85% N₂, for example, at least 90% N₂, or at least 95% N₂, or at least 99% N₂. In certain embodiments as otherwise described herein, the first gaseous composition comprises no more than 10% H₂, for example, no more than 5% H₂, or 3% H₂, or 1% H₂, or 0.1% H₂, or 0.01% H₂. In particular embodiments, the first gaseous composition comprises substantially no H₂.

In certain embodiments as otherwise described herein, the pressure during the contacting of step (i) is in the range of 4 barg to 20 barg, for example in the range of 4 barg to 15 barg, or 4 barg to 12 barg, or 4 barg to 10 barg, or 4 barg to 8 barg, or 5 barg to 20 barg, or 6 barg to 20 barg, or 8 barg to 20 barg, or 10 barg to 20 barg. In certain embodiments as otherwise described herein, the temperature during the contacting of the catalyst during step (i) is no more than 140 °C, or no more than 135 °C. In certain embodiments as otherwise described herein, the temperature during the contacting of the catalyst during step (i) is at least 80 °C, for example, at least 100 °C, or at least 110 °C, or at least 120 °C.

The second gaseous composition is introduced into the reaction zone in order to adjust the H₂:N₂ molar ratio to a desired level. Accordingly, in certain embodiments as otherwise described herein, the second gaseous composition comprises at least 85% H₂, for example, at least 90% H₂, or at least 95% H₂, or at least 99% H₂. In certain embodiments as otherwise described herein, the second gaseous composition comprises no more than 10% N₂, for example no more than 5% N₂, or 3% N₂, or 1% N₂, or 0.1% N₂, or 0.01% N₂. In particular embodiments, the second gaseous composition comprises substantially no N₂.

The H₂/N₂ gaseous composition formed during step (ii) as otherwise described herein may be advantageously adjusted by the second gaseous composition to the desired molar ratio of H₂ and N₂. Accordingly, in certain embodiments as otherwise described herein, the H₂/N₂ gaseous composition formed during step (ii) has a H₂:N₂ molar ratio in the range of 0.3:1 to 2:1 (e.g., in the range of 0.4:1 to 2:1, or 0.5:1 to 2:1, or 0.6:1 to 2:1, or 0.7:1 to 2:1, or 0.8:1 to 2:1, or 0.2:1 to 1.8:1, or 0.2:1 to 1.6:1, or 0.2:1 to 1.4:1, or 0.2:1 to 1.2:1, or 0.4:1 to 1.4:1, or 0.6:1 to 1.4:1, or 0.6:1 to 1.2:1, or 0.8:1 to 1.2:1).

The introduction of the second gaseous composition into the reaction zone may result in an increase in pressure as the additional gas is added. As H₂ is consumed in the reduction process to form water, it can optionally be replenished by addition of further amounts of the second gaseous composition to maintain a target molar ratio of H₂ and N₂. Accordingly, in certain embodiments as otherwise described herein, the pressure resulting from step (ii) is in the range of 12 barg to 30 barg, for example, in the range of 14 barg to 30 barg, or 14 barg to 30 barg, or 14 barg to 24 barg, or 14 barg to 22 barg, or 14 barg to 20 barg, or 20 barg to 30 barg, or 22 barg to 30 barg, or 24 barg to 30 barg, or 24 barg to 28 barg.

During step (iii) as otherwise described herein, in certain embodiments, the pressure may be increased in order to tune the activation conditions. Accordingly, in certain embodiments as otherwise described herein, step (iii) further comprises increasing the pressure to a pressure in the range of 20 barg to 50 barg, for example, in the range of 20 barg to 45 barg, or 20 barg to 40 barg, or 20 barg to 35 barg. The pressure increase may be accomplished through the introduction of the first gaseous composition, the introduction of the second gaseous composition, or a mixture thereof.

The temperature is advantageously ramped at a relatively slow rate in order to protect the catalyst. Accordingly, in certain embodiments as otherwise described herein, the temperature is increased at a ramp rate in the range of 1 °C to 20 °C/hr, for example, in the range of 3 °C to 20 °C/hr, or in the range of 5 °C to 20 °C/hr.

As otherwise described herein, the temperature during step (iii) is ramped to a temperature effective for efficient activation to take place. Accordingly, in certain embodiments as otherwise described herein, the temperature during step (iii) is increased to a range of 230 °C to 260 °C, for example, in the range of 240 °C to 260 °C.

During step (iv) as otherwise described herein, the catalyst is maintained at the final conditions of step (iii) as otherwise described herein for a hold period sufficient for catalyst activation to take place. Accordingly, in certain embodiments as otherwise described herein, the catalyst is maintained at the conditions of step (iii) for a hold period in the range of 2 hr to 96 hr, for example, in the range of 2 hr to 72 hr, or 2 hr to 48 hr, or 3 hr to 96 hr, or 3 hr to 72 hr, or 3 hr to 48 hr, or 4 hr to 72 hr, or 4 hr to 48 hr, or 6 hr to 48 hr.

Subsequent to activation, the catalyst is cooled as part of step (v) as otherwise described herein. In particular embodiments, the catalyst is cooled while maintaining the catalyst in contact with H₂, N₂ or the H₂/N₂ gaseous composition. For example, in certain embodiments as otherwise described herein, the H₂/N₂ gaseous composition of step (v) has a H₂:N₂ molar ratio substantially the same as the H₂:N₂ molar ratio of the H₂/N₂ gaseous composition of step (ii), for example, has a H₂:N₂ molar ratio within 30% (e.g., within 20%, or within 10%) of the H₂:N₂ molar ratio of the H₂/N₂ gaseous composition of step (ii).

As described above, the present inventors have discovered that the gaseous water concentration is an important parameter during activation and must be carefully monitored. This would likely be measured at the reactor exit or the reactor inlet to show water levels given off from the reduction process as well as the effectiveness of the dryer for a recycled gas stream. If the water concentration in the exiting gas is too low, insufficient reduction may be occurring. Conversely, if the water concentration is too high, it can risk damage to the Fischer-Tropsch catalyst. Accordingly, in certain embodiments as otherwise described herein, the process further comprises, during steps (ii)-(v), monitoring the water concentration of the H₂/N₂ gaseous composition. In certain embodiments as otherwise described herein, the process further comprises, if the water concentration exceeds a certain threshold level, adjusting the temperature ramp rate to slow the reduction ramp until water concentrations return to the target range.

After activated and cooling, the catalyst may be contacted in a reaction zone with a gaseous reaction mixture that includes both H₂ and CO in a H₂:CO volume ratio in the range of 1:1 to 3:1. The person of ordinary skill in the art will select a desired ratio, based especially on the H₂:CO volume ratio desired for use in the ultimate FT synthesis process in the steady state. For example, in various embodiments as otherwise described herein, the gaseous reaction mixture comprises H₂ and CO in a H₂:CO volume ratio in the range of 1:1 to 2.5:1, or 1:1 to 2:1, or 1.5:1 to 3:1, or 1.5:1 to 2.5:1, or 1.5:1 to 2:1, or, 2:1 to 3:1, or 2:1 to 3:1. In various embodiments as otherwise described herein, the gaseous reaction mixture comprises H₂ and CO in a H₂:CO volume ratio in the range of 1.5:1 to 2.5:1.

The gaseous reaction mixture may also include other inert gases. For example, the gaseous reaction mixture may include carbon dioxide, methane, N₂ or light hydrocarbon gases in an amount greater than 30%, e.g., greater than 20%, or greater than 10%.

In various embodiments as otherwise described herein, the gaseous reaction mixture comprises at least 20 vol% H₂ and CO (i.e., the proportion of H₂ and CO together is at least 20 vol%). For example, in various embodiments as otherwise described herein, the gaseous reaction mixture comprises at least 30 vol% H₂ and CO (e.g., at least 35 vol% H₂ and CO). In various embodiments as otherwise described herein, the gaseous reaction mixture comprises at least 40% vol% H₂ and CO (e.g., at least 45% H₂ and CO).

Nitrogen gas can be used as an inert gas in the gaseous reaction mixture. In certain embodiments as otherwise described herein, the gaseous reaction mixture further comprises N₂ in an amount up to 80 vol%, e.g., up to 70 vol%, or up to 60 vol%, or up to 50 vol%. For example, in particular embodiments, the gaseous reaction mixture comprises N₂ in an amount in the range of 20 vol% to 80 vol%, e.g., 20 vol% to 70 vol%, or 20 vol% to 60 vol%, or 30 vol% to 80 vol%, or 30 vol% to 70 vol%, or 30% to 60%, or 40 vol% to 80 vol%, or 40 vol% to 70 vol%, or 40 vol% to 60 vol%.

The total amount of reactive feed gases (such as H₂, CO & olefins) in the gaseous reaction mixture of processes as otherwise described herein is desirably high, e.g., at least 90 vol%, or at least 80 vol%, or at least 75 vol%, or at least 70 vol%.

The gaseous reaction mixture may also comprise other gaseous components, such water, methane and other saturated and/or unsaturated light hydrocarbons, preferably being present at a combined concentration of less than 30% by volume.

Upon reaching a temperature at which the Fischer-Tropsch reaction begins (typically around 150-180 °C), a hydrocarbon product composition will begin to form in the reaction zone. And as the Fischer-Tropsch reaches its steady-state operation (e.g., under the conditions of steps (iv) and (v)), hydrocarbon product composition will continue to form, beneficially providing desired products.

Subject to the limitations described herein, the person of ordinary skill in the art can adapt conventional Fischer-Tropsch processes to arrive at the processes of the disclosure. For example, the temperature of the Fischer-Tropsch synthesis can suitably be in the range of 200-400 °C, such as 200-300 °C, or 210-400 °C, or 210-300 °C, or 220-400 °C, or 220-300 °C. In various embodiments as otherwise described herein, the temperature of the Fischer-Tropsch synthesis is in the range of 200-250 °C, e.g., or 200-240 °C, or 200-230 °C, or 200-220 °C, or 210-250 °C, or 210-240 °C, or 210-230 °C, or 220-250 °C, or 220-240 °C, or 230-250 °C. The pressure of the reaction may suitably be in the range from 10-45 barg, e.g., 20-40 barg, or 30-45 barg, or 30-40 barg, or 30-35 barg, or 35-45 barg, or 35-40 barg, or 40-45 barg.

The Fischer-Tropsch synthesis reaction may be performed in any suitable type of reactor, for example it may be performed in a fixed bed reactor, a slurry bed reactor, or a **CANS^{™}** reactor (US8906970, US8722747, US8906970, US10252236, US10654018). Specifically with respect to **CANS** reactors, a passivated catalyst material as described herein can be packaged in a modular catalyst container suitable for use in a reactor tube of the **CANS** reactor (e.g., offsite then transported to the reactor site).

The hydrocarbon composition can vary based on changes in process conditions as known in the art. In certain embodiments, the hydrocarbon composition comprises hydrocarbons (e.g., linear hydrocarbons, branched hydrocarbons, saturated or unsaturated hydrocarbons) and oxygenated derivatives thereof. Examples of the oxygenated derivatives thereof include hydrocarbons with one or more functional group of alcohols, aldehydes, ketones, carboxylic acids, esters, and combinations thereof. In certain embodiments as otherwise described herein, the hydrocarbon composition comprises at least one of alkanes, alkenes, and alcohols.

The person of ordinary skill in the art will adapt suitable catalyst materials for use in the processes described herein. In various desirable embodiments of the processes described herein, the catalyst includes cobalt and optionally one or more other metals or reaction modifiers disposed on a support. Supported cobalt-based materials are well-known in the art, and can generally be adapted for use in the processes and materials described herein.

The catalyst materials described herein include cobalt, in various forms, a support, and optionally other metals or reaction modifiers. Supported cobalt-based materials are well-known in the art, and can generally be adapted for use in the processes and materials described herein.

In various embodiments as otherwise described herein, the catalyst materials as described herein include cobalt in the range of 5 wt% to 35 wt%, calculated as cobalt(0). For example, in certain embodiments as otherwise described herein, cobalt may be present in the range of 7-35 wt%, or 10-35 wt%, or 5-25 wt%, or 7-25 wt%, or 10-25 wt%, or 5-20 wt%, or 7-20 wt%, or 10-20 wt%.

The catalyst materials as described herein can include other metal species, e.g., as promoters. For example, in certain embodiments as otherwise described herein, a catalyst material includes manganese, for example, in an amount in a range of no more than 15 wt%, e.g., no more than 12 wt%, or no more than 10 wt%, or no more than 7 wt%, calculated as manganese(0). In certain such embodiments, a catalyst material includes manganese in an amount in the range of 0.1-15 wt%, e.g., 0.5-12 wt%, or 0.5-10 wt%. Of course, in other embodiments substantially no manganese is present (e.g., less than 0.1 wt% or less than 0.5 wt%) manganese is present.

The processes as described herein efficiently provide means to reduce the Fischer-Tropsch catalyst. Accordingly, in certain embodiments as otherwise described herein, prior to step (i), at least a portion of the cobalt is present as cobalt oxide and/or cobalt hydroxide. For example, the cobalt may be present as cobalt oxide (e.g., CoO, Co₃O₄, or Co₂O₃, or a combination thereof) or cobalt hydroxide (e.g., Co(OH)₂ or Co(OH)₃ or a combination thereof), or a combination of cobalt oxide and cobalt hydroxide. The catalyst can be prepared using methods conventional in the art. In certain embodiments, the cobalt is introduced onto the support through the introduction of a solution containing a soluble cobalt salt (e.g., cobalt nitrate) to the support and the combination calcined and/or oxidized, rendering insoluble cobalt particles (e.g., as cobalt oxide) on the support. For example, the cobalt may be cobalt oxide (e.g., CoO, Co₃O₄, or CO₂O₃, or a combination thereof) or cobalt hydroxide (e.g., Co(OH)₂ or Co(OH)₃ or a combination thereof), or a combination of cobalt oxide and cobalt hydroxide.

In various embodiments of the present disclosure, subsequent to the activation processes described herein at least a portion of the cobalt is present as cobalt (0). Accordingly, in certain embodiments as otherwise described herein, the cobalt of the catalyst after step (v) includes cobalt as cobalt (0) in an amount of at least at least 50%, e.g., at least 75%, or at least 90% with regard to the total amount of cobalt.

Various support materials are known in the art and may be selected based on the precise requirements of the FT reactor or other chemical, mechanical or economic requirements. In certain embodiments as otherwise described herein, the support comprises at least one of titanium oxide, zirconium oxide, cerium oxide, aluminum oxide, silicon oxide and zinc oxide. In particular embodiments, the support comprises exactly one of titanium oxide, zirconium oxide, cerium oxide, aluminum oxide, silicon oxide and zinc oxide. For example, in certain embodiments as otherwise described herein, the support is titanium oxide (titania).

### EXAMPLES

The Examples that follow are illustrative of specific embodiments of the methods of the disclosure, and various uses thereof. They are set forth for explanatory purposes only, and are not to be taken as limiting the scope of the disclosure.

### Example 1

A Fischer-Tropsch reactor was charged with a catalyst comprising 10% Co and 1%Mn on an elemental basis on TiO₂. The catalyst was dried at 130C in N₂ for 2 hrs. Next, N₂ gas was introduced to the reactor to reach an initial pressure of 10 barg. Subsequently, H₂ was introduced to reach a total pressure of 20 barg and a H₂:N₂ molar ratio of 1:1 with a GHSV of 5000 hr⁻¹. The temperature was then increased from an initial temperature of 130 °C to a temperature of 250 °C at a ramp rate of 10 °C/hr and held for 24 hr to form the reduced catalyst. During this time, the water concentration was monitored and maintained below 5000 ppm. The catalyst was then cooled to a temperature of 130 °C and syngas introduced with an H₂:CO molar ratio of 1.8:1. The temperature was ramped up to 220 °C and Fischer-Tropsch synthesis begun.

### Example 2

The activation protocol of Example 1 was repeated except the initial pressure under N₂ was 15 barg, and the total pressure after H₂ introduction was 30 barg.

### Comparative Example 1

The catalyst was reduced by ramping to 1 barg under 100% H₂ and heating to 300 °C for 15 hr, before cooling and subjecting to Fischer-Tropsch synthesis conditions as in Example 1.

The results of Examples 1 and 2, and Comparative Example 1, are summarized in Table 1:

**Table 1**

| | Activation Gas | Activation Temp. | Activation Pressure | FT Temp. | CO Conv. % | C₅+ sel. | CH₄ sel. |
|---|---|---|---|---|---|---|---|
| Comparative Example 1 | 100% H₂ | 300 °C | 1 barg | 220 °C | 60.1 | 87.5 | 6.3 |
| Example 1 | 50%H₂/ 50% N₂ | 250 °C | 20 barg | 220 °C | 56.1 | 88.6 | 5.4 |
| Example 2 | 50%H₂/ 50% N₂ | 250 °C | 30 barg | 220 °C | 60.1 | 88.2 | 5.3 |

As can be seen in Table 1, despite increased % H₂ and increased temperature, which should both aid catalyst activation according to conventional catalyst activation protocols, Comparative Example 1 resulted in undesirably lower C₅₊ selectivity as well as higher CH₄ selectivity compared to Examples 1 and 2. Further, the lower activation temperature of Examples 1 and 2 conserves energy, resulting in lower operating costs.

### Example 3

A Fischer-Tropsch reactor was charged with a catalyst comprising 10% Co and 1%Mn on an elemental basis on TiO₂. The catalyst was dried under N₂ gas for 2 hr at a GHSV of 2000 hr⁻¹ and temperature of 130 °C. Next, N₂ gas was introduced to the reactor to reach an initial pressure of 13 barg. Subsequently, H₂ was introduced to reach a total pressure of 26 barg and a H₂:N₂ molar ratio of 1:1 with a GHSV of 6700 hr⁻¹. The temperature was then increased from an initial temperature of 130 °C to a temperature of 250 °C at a ramp rate of 10 °C/hr and held for 24 hr to form the reduced catalyst. During this time, the water concentration was monitored and maintained below 5000 ppm. The catalyst was then cooled to a temperature of 130 °C and syngas introduced with an H₂:CO molar ratio of 1.8:1 with 51% N₂ and a GHSV of 8795 hr⁻¹. The temperature was ramped from 130 °C to 190 °C at a ramp rate of 10 °C/hr and further from 190 °C to 210 °C at a ramp rate of 1 °C/hr and the pressure increased to 30 barg for Fischer-Tropsch synthesis. Following an allotted time on stream, the temperature was further increased to a second temperature (see Table 2, below).

### Example 4

A Fischer-Tropsch reactor and catalyst was prepared as in Example 3 but with an activation dwell time of 48 hr.

### Example 5

A Fischer-Tropsch reactor and catalyst was prepared as in Example 3 but with an activation pressure of 16 barg, and a GHSV of 4300 hr⁻¹ during activation.

### Example 6

A Fischer-Tropsch reactor and catalyst was prepared as in Example 3 but with an activation pressure of 16 barg, and a GHSV of 4300 hr⁻¹ during activation, and an activation dwell time of 48 hours.

### Example 7

A Fischer-Tropsch reactor and catalyst was prepared as in Example 3 but with an initial activation pressure of 16 barg, and a GHSV of 4300 hr⁻¹ during activation for 24 hours, followed by an increase in the pressure to 26 barg with a GHSV of 6700 hr⁻¹ where the catalyst was held for an additional 24 hr.

### Example 8

A Fischer-Tropsch reactor and catalyst was prepared as in Example 7 but with a temperature ramp rate after drying to 250 °C of 3 °C/hr.

The results of Examples 3-8 are summarized in Table 2, below. Note that the lower Fischer-Tropsch synthesis temperature precludes direct comparison with earlier Examples.

**Table 2**

| | Activation Gas Composition | First Activation Pressure, Time | Second Activation Pressure, Time | First FT Temp., Second FT Temp. | CO Conv. % | C₅+ sel. | CH₄ sel. |
|---|---|---|---|---|---|---|---|
| Ex. 3 | 50%H₂/50% N₂ | 26 barg, 24hr | - | 210 °C | 21.1 | 88.0 | 4.2 |
| | | | | 218 °C | 33.9 | 89.0 | 4.7 |
| Ex. 4 | 50%H₂/50% N₂ | 26 barg, 48hr | - | 210 °C | 22.4 | 88.0 | 4.4 |
| | | | | 217 °C | 33.4 | 88.3 | 4.8 |
| Ex. 5 | 50%H₂/50% N₂ | 16 barg, 24hr | - | 210 °C | 24.6 | 89.1 | 4.2 |
| | | | | 216 °C | 33.9 | 89.5 | 4.3 |
| Ex. 6 | 50%H₂/50% N₂ | 16 barg, 48hr | - | 210 °C | 19.2 | 88.5 | 4.1 |
| | | | | 219 °C | 33.4 | 88.3 | 4.7 |
| Ex. 7 | 50%H₂/50% N₂ | 16 barg, 24hr | 26 barg, 24hr | 210 °C | 22.9 | 88.4 | 4.2 |
| | | | | 218 °C | 35.3 | 88.5 | 5.0 |
| Ex. 8 | 50%H₂/50% N₂ | 16 barg, 24hr, low ramp rate | 26 barg, 24hr | 210 °C | 19.7 | 88.5 | 4.1 |
| | | | | 219 °C | 34.3 | 88.6 | 4.7 |

Various exemplary embodiments of the disclosure include, but are not limited to the enumerated embodiments listed below, which can be combined in any number and in any combination that is not technically or logically inconsistent.
Embodiment 1. A process for the activation of a Fischer-Tropsch synthesis catalyst, the process comprising:
   (i) contacting the catalyst with a first gaseous composition comprising at least 80% N₂ at a pressure in the range of 2 barg to 20 barg at a temperature of no more than 250 °C;
   (ii) introducing a second gas composition to the first gas composition below 175°C where the second gaseous composition comprises at least 80% H₂ to form a H₂/N₂ gaseous composition with a H₂:N₂ molar ratio in the range of 0.2:1 to 2:1, resulting in a pressure in the range of 10 barg to 30 barg;
   (iii) increasing the temperature to a range of 220 °C to 260 °C at a ramp rate of no more than 20 °C/hr, and a GHSV in the range of 2000 hr⁻¹ to 8000 hr⁻¹;
   (iv) maintaining the catalyst at the conditions of step (iii) for a hold period in the range of 2 hr to 96 hr;
   (v) cooling the catalyst to a temperature of no more than 150 °C.
Embodiment 2. The process of embodiment 1, wherein the first gaseous composition comprises no more than 10% H₂ (e.g., no more than 5% H₂, or 3% H₂, or 1% H₂, or 0.1% H₂, or 0.01 % H₂).
Embodiment 3. The process of embodiment 1 or embodiment 2, wherein the first gaseous composition comprises substantially no H₂.
Embodiment 4. The process of any of embodiments 1-3, wherein the second gaseous composition comprises no more than 10% N₂ (e.g., no more than 5% N₂, or 3% N₂, or 1% N₂, or 0.1% N₂, or 0.01% N₂).
Embodiment 5. The process of any of embodiments 1-4, wherein the second gaseous composition comprises substantially no N₂.
Embodiment 6. The process of any of embodiments 1-5, wherein the H₂/N₂ gaseous composition formed during step (ii) has a H₂:N₂ molar ratio in the range of 0.3:1 to 2:1 (e.g., in the range of 0.4:1 to 2:1, or 0.5:1 to 2:1, or 0.6:1 to 2:1, or 0.7:1 to 2:1, or 0.8:1 to 2:1, or 0.2:1 to 1.8:1, or 0.2:1 to 1.6:1, or 0.2:1 to 1.4:1, or 0.2:1 to 1.2:1, or 0.4:1 to 1.4:1, or 0.6:1 to 1.4:1, or 0.6:1 to 1.2:1, or 0.8:1 to 1.2:1).
Embodiment 7. The process of any of embodiments 1-6, wherein the pressure resulting from step (ii) is in the range of 12 barg to 30 barg, for example, in the range of 14 barg to 30 barg, or 16 barg to 30 barg, or 16 barg to 24 barg, or 26 barg to 30 barg.
Embodiment 8. The process of any of embodiments 1-7, wherein the temperature of step (iii) is increased at a ramp rate in the range of 1 °C to 20 °C/hr, for example, in the range of 3 °C to 20 °C/hr, or in the range of 5 °C to 20 °C/hr.
Embodiment 9. The process of any of embodiments 1-8, wherein the temperature during step (iii) is increased to a range of 230 °C to 260 °C, for example, in the range of 240 °C to 260 °C.
Embodiment 10. The process of any of embodiments 1-9, wherein step (iii) further comprises increasing the pressure to a pressure in the range of 30 barg to 50 barg.
Embodiment 11. The process of any of embodiments 1-10, wherein step (v) is performed while maintaining the catalyst in contact with the H₂/N₂ gaseous composition.
Embodiment 12. The process of any of embodiments 1-11, wherein the catalyst comprises cobalt in an amount in the range of 5 wt% to 20 wt% on an elemental basis.
Embodiment 13. The process of embodiment 12, wherein, prior to step (i), at least a portion of the cobalt is cobalt oxide and/or cobalt hydroxide.
Embodiment 14. The process of any of embodiments 1-13, wherein the catalyst comprises manganese in an amount in the range of 0.5 wt% to 10 wt%.
Embodiment 15. The process of any of embodiments 1-14, wherein the catalyst is a supported catalyst, wherein the support is titanium oxide, zirconium oxide, cerium oxide, aluminum oxide, silicon oxide and zinc oxide.
Embodiment 16. The process of embodiment 15, wherein the support is titanium oxide.
Embodiment 17. The process of any of embodiments 1-16, wherein the pressurization is achieved with a compressor, wherein the compressor has a volumetric capacity of at least 1000 Nm³/m3.hr.
Embodiment 18. The process of any of embodiments 1-17, wherein the process further comprises, during steps (ii)-(v), monitoring the water concentration of the H₂/N₂ gaseous composition at the exit of the reactor.
Embodiment 19. The process of embodiment 18, further comprising, if the water concentration exceeds 2000 ppm, adjusting the temperature ramp to dwell or slow the rate to slow the reduction process.
Embodiment 20. The process of any of embodiments 1-19, further comprising performing a Fischer-Tropsch synthesis by contacting the catalyst in a reaction zone with a gaseous reaction mixture comprising H₂ and CO in a ratio in the range of 1:1 to 3:1.
Embodiment 21. The process of embodiment 20, wherein H₂ and CO are present in the gaseous reaction mixture in a H₂:CO ratio in the range of 1:1 to 2.5:1, or 1:1 to 2:1, or 1.5:1 to 3:1, or 1.5:1 to 2.5:1, or 1.5:1 to 2:1, or, 2:1 to 3:1, or2:1 to 3:1.
Embodiment 22. The process of embodiment 20 or embodiment 21, wherein H₂ and CO are present in the gaseous reaction mixture in a H₂:CO ratio in the range of 1.5:1 to 2.5:1.
Embodiment 23. The process of any of embodiments 20-22, wherein the reaction zone is at a temperature of at least 200 °C and a pressure in the range of 10 barg to 45 barg.
Embodiment 24. The process of embodiment 23, wherein the Fischer-Tropsch synthesis is performed at a temperature in the range of 200-250 °C, e.g., or 200-240 °C, or 200-230 °C, or 200-220 °C, or 210-250 °C, or 210-240 °C, or 210-230 °C, or 220-250 °C, or 220-240 °C, or 230-250 °C.
Embodiment 25. The process of embodiment 22 or embodiment 24, wherein the Fischer-Tropsch synthesis is performed at a pressure in the range of 30-45 barg, e.g., 30-40 barg, or 30-35 barg, or 35-45 barg, or 35-40 barg, or 40-45 barg.
Embodiment 26. The process of any embodiments 1 to 25, wherein the activation gases are recycled from the exit of the reactor to the inlet through a gas drying bed, and optionally mixed with fresh gas of composition one or composition two to maintain conditions.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for the activation of a Fischer-Tropsch synthesis catalyst, the process comprising:
(i) contacting the catalyst with a first gaseous composition comprising at least 80% N₂ at a pressure in the range of 2 barg to 20 barg at a temperature of no more than 250 °C;
(ii) introducing a second gas composition to the first gas composition below 175°C where the second gaseous composition comprises at least 80% H₂ to form a H₂/N₂ gaseous composition with a H₂:N₂ molar ratio in the range of 0.2:1 to 2:1, resulting in a pressure in the range of 10 barg to 30 barg;
(iii) increasing the temperature to a range of 220 °C to 260 °C at a ramp rate of no more than 20 °C/hr, and a GHSV in the range of 2000 hr⁻¹ to 8000 hr⁻¹;
(iv) maintaining the catalyst at the conditions of step (iii) for a hold period in the range of 2 hr to 96 hr;
(v) cooling the catalyst to a temperature of no more than 150 °C.

2. The process of claim 1, wherein the first gaseous composition comprises no more than 10% H₂ (e.g., no more than 5% H₂, or 3% H₂, or 1% H₂, or 0.1% H₂, or 0.01% H₂).

3. The process of any of claims claim 1 or claim 2, wherein the second gaseous composition comprises no more than 10% N₂ (e.g., no more than 5% N₂, or 3% N₂, or 1% N₂, or 0.1% N₂, or 0.01% N₂).

4. The process of any of claims 1-3, wherein the H₂/N₂ gaseous composition formed during step (ii) has a H₂:N₂ molar ratio in the range of 0.3:1 to 2:1 (e.g., in the range of 0.4:1 to 2:1, or 0.5:1 to 2:1, or0.6:1 to 2:1, or0.7:1 to 2:1, or0.8:1 to 2:1, or0.2:1 to 1.8:1, or0.2:1 to 1.6:1, or 0.2:1 to 1.4:1, or 0.2:1 to 1.2:1, or 0.4:1 to 1.4:1, or 0.6:1 to 1.4:1, or 0.6:1 to 1.2:1, or 0.8:1 to 1.2:1).

5. The process of any of claims 1-4, wherein the temperature during step (iii) is increased to a range of 230 °C to 260 °C, for example, in the range of 240 °C to 260 °C.

6. The process of any of claims 1-5, wherein step (iii) further comprises increasing the pressure to a pressure in the range of 30 barg to 50 barg.

7. The process of any of claims 1-6, wherein step (v) is performed while maintaining the catalyst in contact with the H₂/N₂ gaseous composition.

8. The process of any of claims 1-7, wherein the catalyst comprises cobalt in an amount in the range of 5 wt% to 20 wt% on an elemental basis.

9. The process of claim 8, wherein, prior to step (i), at least a portion of the cobalt is cobalt oxide and/or cobalt hydroxide.

10. The process of any of claims 1-9, wherein the catalyst comprises manganese in an amount in the range of 0.5 wt% to 10 wt%.

11. The process of any of claims 1-10, wherein the catalyst is a supported catalyst, wherein the support is titanium oxide, zirconium oxide, cerium oxide, aluminum oxide, silicon oxide and zinc oxide.

12. The process of claim 11, wherein the support is titanium oxide.

13. The process of any of claims 1-12, wherein the pressurization is achieved with a compressor, wherein the compressor has a volumetric capacity of at least 1000 Nm³/m3.hr.

14. The process of any of claims 1-13, wherein the process further comprises, during steps (ii)-(v), monitoring the water concentration of the H₂/N₂ gaseous composition at the exit of the reactor.

15. The process of claim 14, further comprising, if the water concentration exceeds 2000 ppm, adjusting the temperature ramp to dwell or slow the rate to slow the reduction process.

16. The process of any of claims 1-15, further comprising performing a Fischer-Tropsch synthesis by contacting the catalyst in a reaction zone with a gaseous reaction mixture comprising H₂ and CO in a ratio in the range of 1:1 to 3:1.

17. The process of claim 16, wherein the reaction zone is at a temperature of at least 200 °C and a pressure in the range of 10 barg to 45 barg.

18. The process of any claims 1 to 17, wherein the activation gases are recycled from the exit of the reactor to the inlet through a gas drying bed, and optionally mixed with fresh gas of composition one or composition two to maintain conditions.
